# EUROPÄISCHE PATENTSCHRIFT

(11) **EP 0 565 718 B1**
(45) Veröffentlichungstag und Bekanntmachung des Hinweises auf die Patenterteilung: **25.06.1997**
(21) Anmeldenummer: 91919158.5
(22) Anmeldetag: 06.11.1991
(51) Int. Cl.: A61F 13/15, B29C 51/00

(54) **FLEXIBLE KUNSTSTOFFOLIE MIT RIPPENSTRUKTUR**
FLEXIBLE PLASTIC SHEET HAVING RIB STRUCTURE
FEUILLE PLASTIQUE SOUPLE PRESENTANT UNE STRUCTURE NERVUREE

(43) Veröffentlichungstag der Anmeldung: 20.10.1993
(73) Patentinhaber: UNI-CHARM COMPANY LIMITED, Kawanoe-Shi, Ehime 799-01 (JP)
(72) Erfinder: YAMAMOTO, Masamitsu, Ehime 799-01 (JP); YAGI, Yagoro, Shiga 520 (JP); MURAKAMI, Masaki, Kawanoe-shi Ehime 799-01 (JP)
(74) Vertreter: Sperling, Rüdiger, Dipl.-Ing.
(86) Internationale Anmeldenummer: JP9101521
(87) Internationale Veröffentlichungsnummer: WO9308778

(56) Entgegenhaltungen:
- EP-A- 0 313 766
- WO-A-91/03367
- JP-A-58 019 252
- JP-A-62 057 975
- JP-A-62 161 364

## Beschreibung

Die vorliegende Erfindung betrifft eine flexible Kunststoffolie mit einem gewebeähnlichen Erscheinungsbild, die zur Verwendung als flüssigkeitsundurchlässige Außenlage geeignet ist, die einen der wesentlichen Bestandteile eines am Körper zu tragenden Wegwerfhygieneartikels bildet.

Flexible Kunststoffolien einschließlich thermoplastischer Folien haben gewöhnlich einen für herkömmliche Kunststoffolien kennzeichnenden, in gewisser Weise abstoßenden Glanz und fühlen sich klebrig an, was bei den Benutzern bei einigen Anwendungsgebieten derartiger Folien auf Ablehnung stößt. Um einen derartigen Glanz zu mildern und den Griff der Folie zu verbessern, sind bereits mehrere Techniken bekannt, beispielsweise eine Technik, durch die die Folienoberfläche geprägt wird, um einen derartigen Glanz wegzunehmen und gleichzeitig die Oberfläche mit Unregelmäßigkeiten zu versehen, und eine Technik, durch die ein dritter Bestandteil vorab dem Rohmaterial für die Folie beigemischt wird, so daß die gewünschte Abmilderung des Glanzes und die Verbesserung des Griffes in einem Schritt der Folienherstellung erzielt werden kann.

Diese bekannten Techniken sind jedoch bisher nicht in der Lage, eine angemessene Verbesserung von Kunststoffolien zu erzielen, die insbesondere als Oberflächenlage von am Körper zu tragenden Wegwerfhygieneartikeln, wie etwa Damenbinden und Wegwerfwindeln verwendet werden, da der Glanz und der Griff der Kunststoffolie bei den Benutzern derartiger am Körper zu tragender Artikel entschieden abgelehnt werden. Das US Patent Nr. 4,342,314 zeigt eine Technik zum Versehen einer flexiblen Kunststoffolie mit einem faserigen Erscheinungsbild und einer Kapillarstruktur zur Verbesserung des Erscheinungsbildes wie auch des Griffes auf. Gemäß der durch dieses Patent aufgezeigten Technik kann ein Erscheinungsbild erzielt werden, das einem gewebten Stoff stark ähnelt, wobei jedoch dessen Oberfläche relativ glatt sein kann und daher dazu neigt, sogar nach der Behandlung der Folie gemäß der aufgezeigten Technik noch einen abstoßenden Glanz aufzuweisen.

Demgemäß ist es eine Hauptaufgabe der vorliegenden Erfindung, eine flexible Kunststoffolie auf ihrer Oberfläche mit einer vielzahl von einander überkreuzenden Rippen zu versehen, um so ein gewebtem Stoff ähnliches Erscheinungsbild zu schaffen und dadurch den unerwünschten Glanz abzumildern und den Griff zu verbessern, der herkömmlicherweise allgemein für Kunststoffolien kennzeichnend ist.

Die vorstehend dargelegte Aufgabe wird gemäß vorliegender Erfindung durch eine flexible Kunststoffolie mit einer Rippenstruktur erzielt, die ein gewebeähnliches Erscheinungsbild aufweist, umfassend
eine Vielzahl von in einer ersten Richtung verlaufenden Rippen, die jeweils einander gegenüberliegende, nach unten gekrümmte Seitenränder aufweisen, und eine Vielzahl von in einer zweiten Richtung verlaufenden Rippen, die jeweils einander gegenüberliegende, nach unten gekrümmte Seitenränder aufweisen, so daß diese Rippen erster Richtung und Rippen zweiter Richtung einander überkreuzen;
einen von einem jeweiligen Paar benachbarter Rippen erster Richtung und einem jeweiligen Paar benachbarter Rippen zweiter Richtung, das das Paar benachbarter Rippen erster Richtung überkreuzt, begrenzten, durchgehenden Bereich; und

Oberseiten des Paares der benachbarten Rippen zweiter Richtung, die einerseits mit den Seitenrändern des Paares benachbarter Rippen erster Richtung an ersten und zweiten Kreuzungspunkten verbunden sind, die benachbart und einander diagonal gegenüberliegend angeordnet sind, und Oberseiten des Paares benachbarter Rippen erster Richtung, die andererseits mit den Seitenrändern des Paares benachbarter Rippen zweiter Richtung an dritten und vierten Kreuzungspunkten, die benachbart und einander diagonal gegenüberliegend angeordnet sind, verbunden sind.

Dabei schließt das Paar benachbarter Rippen erster Richtung vorzugsweise Brücken ein, die einander gegenüberliegende untere Ränder dieser Rippen verbinden.

Gemäß einen weiteren Aspekt der Erfindung ist diese Kunststoffolie vorzugsweise flüssigkeitsundurchlässig.

Die durch die Erfindung erzielten vorteilhaften Auswirkungen sind ohne weiteres aus der nachstehenden Beschreibung ersichtlich.

Bei der flexiblen Kunststoffolie gemäß der Erfindung mit vorstehend beschriebenem Aufbau überkreuzen die Rippen erster Richtung die Rippen zweiter Richtung und an jedem Kreuzungspukt ist eine der Rippen nach oben vorgewölbt, während die andere Rippe abgesenkt ist, so daß die Oberseite der letzteren mit den Seitenrändern ersterer verbunden ist und dadurch ein Erscheinungsbild ähnlich gewebtem Stoff, wie etwa Gewebe mit Leinwandbindung oder Gewebe mit Köperbindung geschaffen wird. Diese Kunststoffolie ist in den durch die Rippen erster Richtung und Rippen zweiter Richtung umgebenen Bereichen durchgehend, so daß somit eine flexible, flüssigkeitsundurchlässige Kunststoffolie geschaffen wird.

Nachfolgend wird die Erfindung anhand von Figuren näher erläutert. Es zeigen:
Fig. 1 und 2 eine perspektivische Ansicht bzw. Draufsicht einer Ausführungsform der flexiblen Kunststoffolie, die gemäß der Lehre der vorliegenden Erfindung aufgebaut ist;
Fig. 3 eine Draufsicht auf eine weitere Ausführungsform;
Fig. 4 und 5 eine Draufsicht bzw. Schnittdarstellung einer weiteren Ausführungsform;
Fig. 6 eine Draufsicht einer weiteren Ausführungsform; und
Fig. 7 eine perspektivische Darstellung der Kunststoffolie gemäß der Erfindung, die beispielhaft als Außenlage einer Damenbinde verwendet ist.

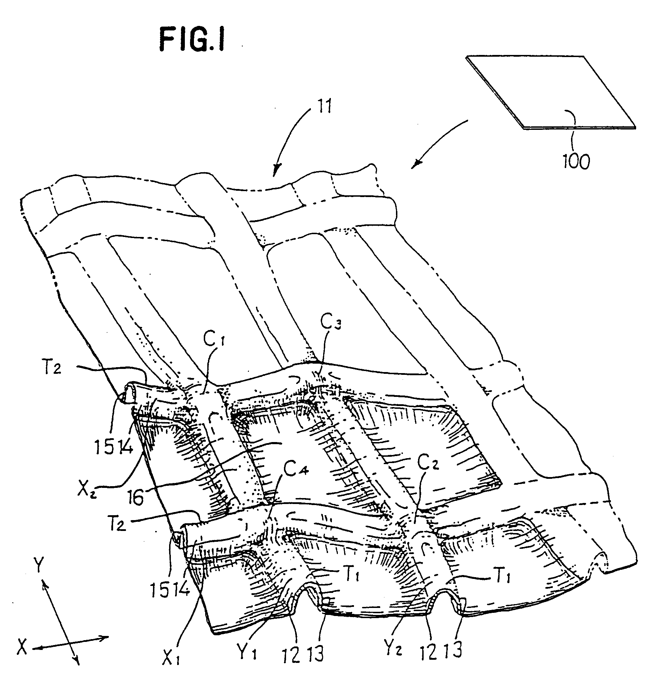

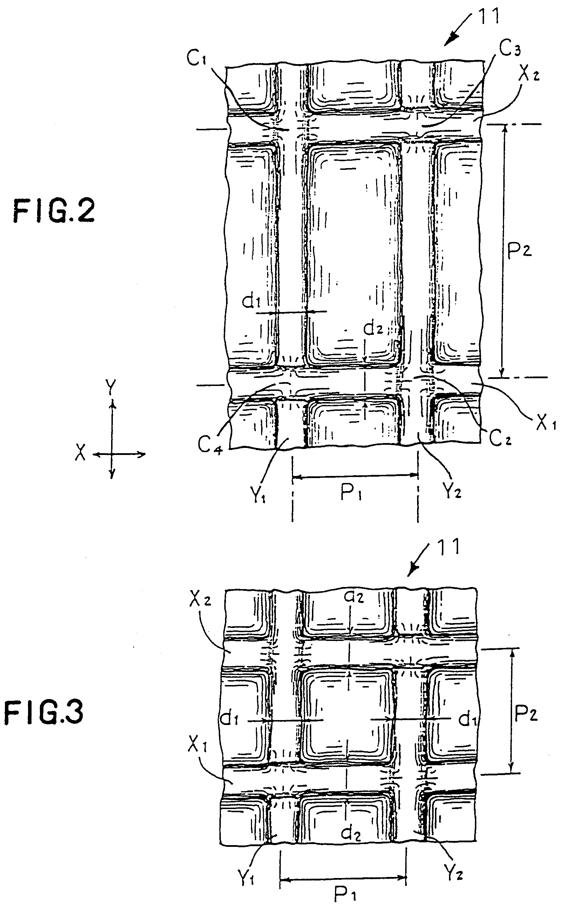

In Fig. 1 und 2 ist eine Ausführungsform einer gemäß der Lehre der Erfindung aufgebauten flexiblen Kunststoffolie 11 mit Rippenstruktur in perspektivischer Ansicht bzw. in Draufsicht dargestellt.

Wie insbesondere in Fig. 1 zu erkennen ist, weist die durch Thermoformung einer thermoplastischen Folie 100 erhaltene Folie 11 eine Struktur auf, die eine Vielzahl von Rippen und von diesen Rippen begrenzte Flächen umfaßt, wie teilweise durch ausgezogene Linien dargestellt. Eine derartige Struktur kann sich sowohl in Längs- als auch in Querrichtung wiederholend erstrecken, wie durch unterbrochene Linien angedeutet, um die vollständige Folie 11 zu erhalten. Die Folie 11 umfaßt Rippen Y₁, Y₂ ... Yₙ, die in einer ersten Richtung Y verlaufen, und Rippen X₁, X₂ ... Xₙ, die in einer zweiten Richtung X verlaufen, wobei diese Rippen eine im wesentlichen gleichförmige Breite aufweisen. Wie in Fig. 1 dargestellt, sind die jeweiligen Rippen in Querrichtung nach unten gekrümmt, so daß die Rippen Y₁, Y₂ jeweils zwei untere Ränder 12, 13 aufweisen, die in Längsrichtung parallel zueinander verlaufen, und die Rippen X₁, X₂ in ähnlicher Weise jeweils zwei untere Ränder 14, 15 aufweisen, die parallel zueinander verlaufen. Das in der ersten Richtung verlaufende Paar von benachbarten Rippen Y₁, Y₂ überkreuzt das Paar von benachbarten Rippen X₁, X₂, die in der zweiten Richtung verlaufen, an Kreuzungspunkten C₁ bis C₄. An ersten und zweiten Kreuzungspunkten C₁, C₂, die benachbart und einander diagonal gegenüberliegend angeordnet sind, senken sich die jeweiligen Oberseiten T₂ der Rippen zweiter Richtung X₁, X₂ ab und sind mit den jeweiligen Seitenrändern 12, 13 der Rippen erster Richtung Y₁, Y₂ verbunden, während an den dritten und vierten Kreuzungspunkten C₃, C₄, die ebenfalls benachbart und einander diagonal gegenüberliegend angeordnet sind, die jeweiligen Oberseiten T₁ der Rippen erster Richtung Y₂, Y₁ sich absenken und mit den jeweiligen Seitenrändern 14, 15 der Rippen zweiter Richtung X₁, X₂ verbunden sind. Das Rippenpaar Y₁, Y₂ überkreuzt das Rippenpaar X₁, X₂ im wesentlichen im rechten Winkel und die Folie 11 ist in jeder der von diesen beiden Rippenpaaren umgebenen Flächen 16 durchgehend. Als Folge schaffen die jeweiligen Rippen gemeinsam ein einem Gewebe mit Leinenbindung ähnliches Erscheinungsbild und die Folie 11 ist bedingt durch ihre zusammenhängende Ausführung allgemein flüssigkeitsundurchlässig.

Fig. 2 ist eine Draufsicht auf die in Fig. 1 gezeigte Folie. Wie in dieser Figur dargestellt, haben die Rippen erster Richtung Y₁, Y₂ einen Abstand P₁ und die Rippen zweiter Richtung einen Abstand P₂, wobei P₁ < P₂. Die Folie 11 kann jedoch so aufgebaut sein, daß eine Beziehung von P₁ ≥ P₂ entsteht. Darüberhinaus können die Rippen einander schräg überkreuzen anstatt sich im rechten Winkel zu überkreuzen.

Die Rippen Y₁, Y₂ haben eine Breite d₁ und die Rippen X₁, X₂ eine Breite d₂. Während diese bestimmte Ausführungsform so dargestellt ist, daß die Breite d₁ im wesentlichen gleich der Breite d₂ ist, kann einer dieser Werte d₁ und d₂ größer als der andere sein und jede Rippe kann entlang ihrem in Längsrichtung verlaufenden Mittelabschnitt örtlich verjüngt sein.

Damit die Folie 11 ein einem gewebten Stoff stark ähnelndes Erscheinungsbild aufweist, sollte die Rippenbreite d₁, d₂ 0,01 bis 3 mm betragen, vorzugsweise 0,05 bis 2 mm, und die Anzahl der von den Rippen begrenzten Flächen 16 sollte 5 bis 90 pro 25,4 mm, vorzugsweise 10 bis 60 pro 25,4 mm der jeweiligen Rippen betragen. Die Folie 11 kann aus einer Folie 100 hergestellt sein, die 5 bis 50 g/m², vorzugsweise 20 bis 30 g/m² Polyethylen, Polypropylen und anderes thermoplastisches Material, vorzugsweise ein hydrophobes thermoplastisches Material umfaßt. Die Verwendung einer derartigen Folie 100 versetzt die Folie 11 in die Lage, einen gewünschten Grad von Flexibilität zu erhalten. Es versteht sich, daß der Begriff "Flexibilität" die Flexibilität bezeichnet, die für eine flüssigkeitsundurchlässige Lage eines am Körper zu tragenden Wegwerfhygieneartikels erforderlich ist, eine Anwendung, für die die Folie 11 besonders geeignet ist. Eine Abmilderung des in gewisser Weise abstoßenden Glanzes, der herkömmliche Kunststoffolien kennzeichnet, eine Verbesserung des Griffs und eine weitere Hervorhebung des gewebeähnlichen Erscheinungsbildes der erhaltenen Folie 11 kann in wirksamer Weise durch verschiedene Gegenmaßnahmen erzielt werden, beispielsweise durch Aufrauhen der Rippenoberfläche zur Förderung einer diffusen Reflexion des einfallenden Lichtes, unregelmäßiges Verändern der Breite jeder Rippe, Anordnung der Rippen in ungleichmäßigen Abständen, geeignete Färbung der Folie 100 und Mischung der Folie 100 nach einer Formel, die einen Glanzverlust durch die Thermoformung bewirkt.

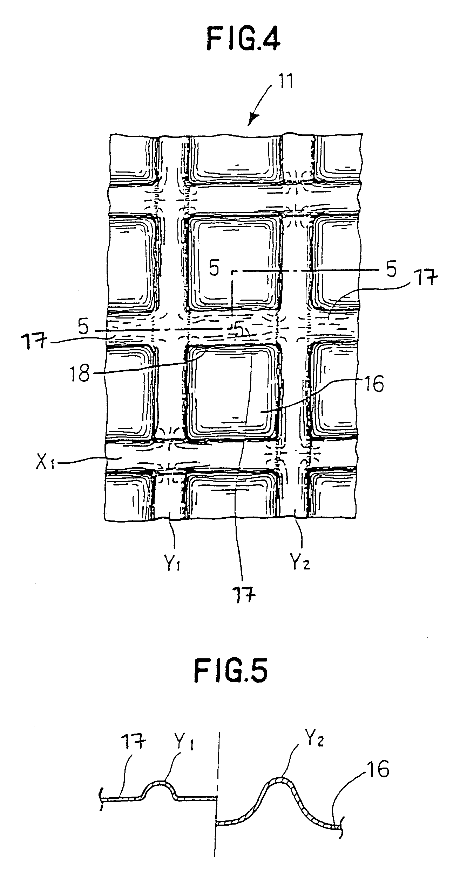

Fig. 3 ist eine Draufsicht auf eine weitere Ausführungsform der Erfindung. Aus dieser Figur ist ersichtlich, daß die Folie 11 Rippen Y₁, Y₂ ... X₁, X₂ ... mit im wesentlichen gleichförmigen Breiten d₁, d₂ umfaßt, die in gleichmäßigen Abständen P₁, P₂ angeordnet sind und somit das Erscheinungsbild eines Gewebes mit Leinwandbindung darstellen.

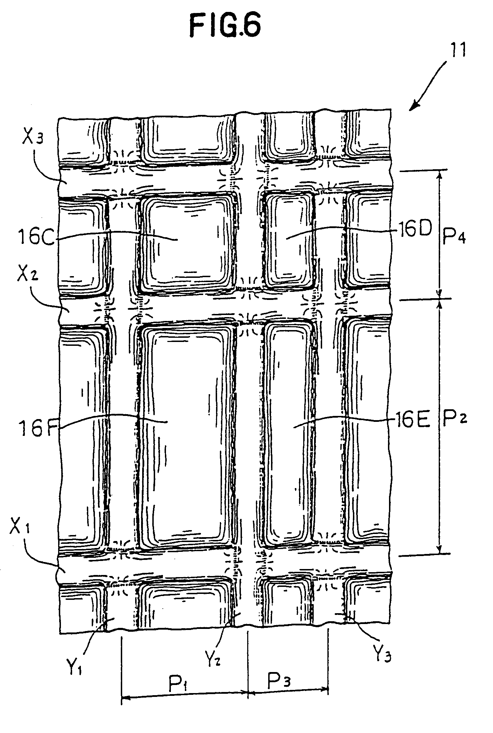

In Fig. 4 und 5 ist eine weitere Ausführungsform der Erfindung in Draufsicht bzw. einer Schnittdarstellung entlang einer Linie 5-5 in Fig. 4 dargestellt. Wie hier gezeigt, ist zwischen den Rippen Y₁, Y₂ in dieser Folie 11 eine rippenartige Brücke 17 vorgesehen, die diese Rippen Y₁, Y₂ miteinander verbindet. Jede Brücke hat eine Oberseite, die niedriger ist als die Oberseite T₁ der Rippen Y₁ und Y₂ und die mit den jeweiligen Seitenrändern 12, 13 dieser Rippen Y₁, Y₂ verbunden ist. Diese Art von Brücke 17 kann nach Wunsch zwischen den jeweils paarweise verlaufenden benachbarten Rippen vorgesehen sein. Wenn die Folie 11 derartige Brücken 17 enthält, wirken die Brücken 17 mit den benachbarten Rippen zusammen um ein Erscheinungsbild von mit Köperbindung gewebtem Stoff zu schaffen.

Fig. 6 zeigt noch eine weitere Ausführungsform der Erfindung in Draufsicht. Diese Folie 11 umfaßt Rippen erster Richtung Y₁, Y₂, Y₃ ..., die in Abständen P₁, P₃ angeordnet sind, und Rippen zweiter Richtung X₁, X₂, X₃ ..., die in Abständen P₂, P₄ angeordnet sind, wobei diese Abstände sich voneinander unterscheiden. Die Rippen begrenzen Flächen 16C bis 16F und in der vollständigen Folie 11 sind die Abstände P₁ und P₃ der Rippen erster Richtung (Y₁, Y₂, Y₃) abwechselnd wiederholt und die Abstände P₂ und P₄ der Rippen zweiter Richtung (X₁, X₂, X₃) sind ebenfalls abwechselnd wiederholt. In dieser Ausführungsform der Folie 11 kann auch die Rippenbreite auf Wunsch variiert werden und zwischen den jeweiligen paarweise verlaufenden benachbarten Rippen können ebenso wie in der in Fig. 4 gezeigten Ausführungsform Brücken vorgesehen sein.

Während die vorstehend beschriebenen Ausführungsformen der Folie 11 durch verschiedene Verfahren erhalten werden können, umfaßt eines der relativ einfachen Verfahren das Auflegen der Folie 100 in thermisch erweichtem Zustand auf die Oberseite eines Drahtnetzes mit einer einem gewebtem Stoff entsprechenden Oberflächenstruktur, wie zum Beispiel Gewebe mit Leinwandbindung, Köperbindung oder Satinbindung, oder das thermische Erweichen der Folie 100 nach dem Auflegen auf die Oberseite des Drahtnetzes und das Beaufschlagen der Folie 100 mit einer gemäßigten Vakuumsaugwirkung, die so gesteuert wird, daß die Folie 100 nicht reißt, und von der Unterseite des Drahtnetzes angelegt wird. Die Berührung der Folie 100 mit der Drahtnetzstruktur unter Vakuumsaugwirkung, gefolgt vom raschen Abkühlen der Folie 100, erlaubt es, diese Folie gemäß einem Muster der Drahtnetzstruktur zur fertigen Folie 11 thermisch zu formen. Die Unterseite der Folie 11, wie in Fig. 1 dargestellt, ist die gegen das Drahtnetz gedrückte Oberfläche. Zur kontinuierlichen Herstellung der Folie 11 kann eine drehbare Walze an ihrem Umfang mit dem Drahtnetz versehen sein, wobei die drehbare Walze einer geeigneten Saugwirkung unterworfen wird und eine Rolle der Folie 100 kontinuierlich auf die Walze zur Thermoformung abgewickelt wird. Das Drahtnetz kann wenigstens teilweise aus Drahtlitze gebildet sein, um auf den Rippenoberflächen eine Unregelmäßigkeit entsprechend der Drahtlitze zu erzeugen.

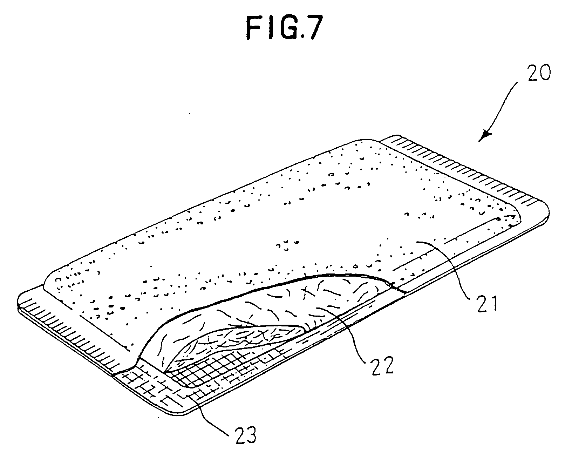

Fig. 7 zeigt eine Damenbinde 20, in der die Folie 11 als Außenlage 23 verwendet wird, in teilweise ausgebrochener perspektivischer Darstellung. Die Binde 20 umfaßt einen Absorptionskern 22, der zum Aufnehmen und Festhalten von Menstruationsflüssigkeit eingerichtet ist, eine luft- und flüssigkeitsdurchlässige Decklage 21 und die flüssigkeitsundurchlässige Außenlage 23. Die Außenlage 23 umfaßt die in Fig. 1 dargestellte Folie 11 mit nach außen gerichteten Rippenoberseiten, der Absorptionskern 22 umfaßt eine Mischung von Faserpulpe und hoch wasserabsorbierendem Polymerpulver und die Decklage 21 umfaßt poröse Polyethylenfolie. Die Decklage 21 und die Außenlage 23 sind entlang den vier Rändern der Binde 20 miteinander verbunden, so daß keine Menstruationsflüssigkeit austritt.

Die gemäß der Lehre der vorliegenden Erfindung aufgebaute flexible Kunststoffolie ist industriell als flüssigkeitsundurchlässige Außenlage eines am Körper zu tragenden Wegwerfartikels unter folgenden Gesichtspunkten verwendbar.
1. Die Folie weist feine Vorwölbungen und Wellenformen auf ihrer Oberfläche auf, an denen die diffuse Reflexion einfallenden Lichtes auftritt, um einen für herkömmliche Kunststoffolien kennzeichnenden, in gewissem Maße abstoßenden Glanz abzumildern, und die eine mit der Haut des Trägers in Berührung stehende Oberfläche verringern, um so den in gewisser Weise klebrigen Griff zu vermeiden, der ebenfalls für herkömmliche Kunststoffolien kennzeichnend ist.
2. Die Folie weist ein der Struktur von Gewebe, beispielsweise mit Leinwandbindung, Köperbindung oder ähnlichem entsprechendes Erscheinungsbild in Abhängigkeit vom Muster der Rippenanordnung auf.
3. Durch das Fehlen des klebrigen Griffes und durch die Flüssigkeitsundurchlässigkeit kann die Folie als auslaufsichere Lage für am Körper zu tragende Wegwerfhygieneartikel, wie zum Beispiel Damenbinden und Wegwerfwindeln verwendet werden.

## Patentansprüche

1. Flexible Kunststoffolie mit einer Rippenstruktur und einem Erscheinungsbild ähnlich gewebtem Stoff, umfassend:
eine Vielzahl von in einer ersten Richtung (Y) verlaufenden Rippen (Y₁, Y₂), die jeweils einander gegenüberliegende, nach unten gekrümmte Seitenränder (12,13) aufweisen, und eine Vielzahl von in einer zweiten Richtung (X) verlaufenden Rippen (X₁, X₂), die jeweils einander gegenüberliegende, nach unten gekrümmte Seitenränder (14,15) aufweisen, so daß diese Rippen erster Richtung (Y₁, Y₂) und Rippen zweiter Richtung (X₁, X₂) einander überkreuzen;
einen von einem jeweiligen Paar benachbarter Rippen erster Richtung (Y₁, Y₂) und einem jeweiligen Paar benachbarter Rippen zweiter Richtung (X₁, X₂), das das Paar benachbarter Rippen erster Richtung (Y₁, Y₂) überkreuzt, begrenzten, durchgehenden Bereich (16); und
Oberseiten (T₂) des Paares der benachbarten Rippen zweiter Richtung (X₁, X₂), die einerseits mit den Seitenrändern (12,13) des Paares benachbarter Rippen erster Richtung (Y₁, Y₂) an ersten und zweiten Kreuzungspunkten (C₁, C₂) verbunden sind, die benachbart und einander diagonal gegenüberliegend angeordnet sind, und Oberseiten (T₁) des Paares benachbarter Rippen erster Richtung (Y₁, Y₂), die andererseits mit den Seitenrändern (14,15) des Paares benachbarter Rippen zweiter Richtung (X₁, X₂) an dritten und vierten Kreuzungspunkten (C₃, C₄), die benachbart und einander diagonal gegenüberliegend angeordnet sind, verbunden sind und
wobei die beiden benachbarten Rippen erster Richtung (Y₁, Y₂) Brücken (17) einschließen, die einander gegenüberliegende untere Ränder (12,13) dieser Rippen verbinden und deren Oberseiten niedriger sind als die Oberseiten (T₁,T₂) der Rippen (Y₁, Y₂).

2. Flexible Kunststoffolie nach Anspruch 1,
wobei die flexible Kunststoffolie (11) flüssigkeitsundurchlässig ist.

## Claims

1. A flexible plastics sheet with a rib structure and an appearance like that of woven material, comprising:
a plurality of ribs (Y₁, Y₂) running in a first direction (Y), which each have downwardly curved side edges (12, 13) opposite one another, and a plurality of ribs (X₁, X₂) running in a second direction (X) and which each have downwardly curves side edges (14, 15) opposite one another, so that these ribs of with the first direction (Y₁, Y₂) and ribs with the second direction (X₁, X₂) cross one another;
a continuous regions (16) bounded in each case by a respective pair of adjacent ribs with the first direction (Y₁, Y₂) and a respective pair of adjacent ribs with the second direction (X₁, X₂) which cross the pair of adjacent ribs with the first direction (Y₁, Y₂); and
upper sides (T₂) of the pair of adjacent ribs with the second direction (X₁, X₂) which are connected on the one hand to the side edges (12, 13) of the pair of adjacent ribs with the first direction (Y₁, Y₂) at first and second crossing points (C₁, C₂) which are adjacent and diagonally opposite one another, and upper sides (T₁) of the pair of adjacent ribs with the first direction (Y₁, Y₂) which are connected on the other hand to the side edges (14, 15) of the pair of adjacent ribs with the second direction (X₁, X₂) at third and fourth crossing points (C₃, C₄) which are adjacent and diagonally opposite one another, and
wherein the two adjacent ribs with the first direction (Y₁, Y₂) comprise bridges (17) which connect lower edges (12, 13) of these ribs opposite one another and whose upper sides are lower than the upper sides (T₁, T₂) of the ribs (Y₁, Y₂).

2. A flexible plastics sheet according to claim 1, wherein the flexible plastics sheet (11) is impermeable to liquids.

## Revendications

1. Feuille de matière plastique souple présentant une structure à côtes et offrant un aspect semblable à celui de l'étoffe tissée, comprenant :
- une pluralité de côtes (Y₁, Y₂) orientées dans un premier sens (Y) présentant, respectivement, des bords latéraux (12, 13) opposés entre eux et s'incurvant vers le bas, et une pluralité de côtes (X₁, X₂) orientées dans un second sens (X) présentant, respectivement, des bords latéraux (14, 15) opposés entre eux et s'incurvant vers le bas, ces côtes de premier sens (Y₁, Y₂) et ces côtes de second sens (X₁, X₂) s'entrecroisant;
- une zone continue (16) délimitée par deux côtes voisines de premier sens (Y₁, Y₂) et deux côtes voisines de second sens (X₁, X₂) qui croisent lesdites deux côtes voisines de premier sens (Y₁, Y₂); et
- des faces supérieures (T₂) des côtes voisines de second sens (X₁, X₂), qui sont jointes, d'une part, aux bords latéraux (12, 13) des côtes voisines de premier sens (Y₁, Y₂) au niveau de premier et de second points de croisement (C₁, C₂), qui sont positionnés au voisinage l'un de l'autre et en opposition diagonale mutuelle, et des faces supérieures (T₁) des côtes voisines de premier sens (Y₁, Y₂) jointes, d'autre part, aux bords latéraux (14, 15) des côtes voisines de second sens (X₁, X₂) au niveau de troisième et de quatrième points de croisement (C₃, C₄), qui sont positionnés au voisinage l'un de l'autre et en opposition diagonale mutuelle, et
- les côtes voisines de premier sens (Y₁, Y₂) incluent des ponts (17) qui relient des bords inférieurs (12, 13) de ces côtes, mutuellement opposés, et dont les faces supérieures sont plus basses que les faces supérieures (T₁, T₂) des côtes (Y₁, Y₂).

2. Feuille de matière plastique souple selon la revendication 1, cette feuille de matière plastique souple (11) étant imperméable aux liquides.
